# FASCICULE DE BREVET EUROPEEN

(11) **EP 4 106 668 B1**
(45) Date de publication et mention de la délivrance du brevet: **19.11.2025**
(21) Numéro de dépôt: 21709082.8
(22) Date de dépôt: 11.02.2021
(51) Int. Cl.: A61F 2/00, A61F 2/50

(54) **ENVELOPPE D'HABILLAGE POUR PROTHESE**
ABDECKSCHALE FÜR EINE PROTHESE
COVERING SHELL FOR A PROSTHESIS

(30) Priorité: 19.02.2020 FR 2001627
(43) Date de publication de la demande: 28.12.2022
(73) Titulaire: Ottobock Réseau Orthopédie & Services, 91940 Les Ulis (FR)
(72) Inventeur: SOUPLY, Marc, 38220 VIZILLE (FR); KHOUNLAVONG, Anousak, 38170 SEYSSINET-PARISET (FR); RONAYETTE-LAMOINE, Elodie, 38100 GRENOBLE (FR); REVAIS, Jules, 38170 SEYSSINET-PARISET (FR)
(74) Mandataire: Santarelli
(86) Numéro de dépôt international: PCT/FR2021/050249
(87) Numéro de publication internationale: WO 2021/165602

(56) Documents cités:
- EP-A1- 2 944 290
- CN-A- 1 980 616
- US-B2- 10 172 726

## Description

### DOMAINE TECHNIQUE ET ÉTAT DE LA TECHNIQUE ANTÉRIEURE

La présente invention se rapporte à une enveloppe d'habillage pour prothèse.

Suite à une amputation d'un membre, pour retrouver sa mobilité, un patient se trouve habituellement appareillé avec une prothèse qui va partiellement remplacer la fonctionnalité du membre perdu. La forme physique d'une telle prothèse est déterminée par les contraintes imposées par sa fonction. Par exemple, pour obtenir une prothèse de remplacement de la jambe, la prothèse comprend une barre se terminant par une articulation mécanique afin d'assumer aussi bien que possible une fonctionnalité de jambe. L'aspect physique de la prothèse reste d'importance secondaire.

Afin de conférer une esthétique similaire au membre perdu, une prothèse peut être parée d'une enveloppe d'habillage. Un exemple d'une telle enveloppe est décrit par le document EP 2 944 290 A1. Pour obtenir un aspect naturel, l'enveloppe décrite par le document cité emprunte la forme d'une jambe. De plus, des ouvertures sont prévues pour permettre une aération et pour réduire le poids de l'enveloppe.

WO 2017/012888 décrit une structure exosquelettique selon le préambule de la revendication 1.

Les enveloppes connues dans l'art antérieur ne reproduisent qu'imparfaitement un membre remplacé.

### EXPOSÉ DE L'INVENTION

Le but de la présente invention est alors d'offrir une enveloppe d'habillage pour prothèse d'un membre donné qui reproduit de manière plus réaliste le membre remplacé.

Le but énoncé ci-dessus est atteint par une enveloppe qui ressemble, au toucher, au membre remplacé. L'enveloppe de la présente invention ressemble par sa flexibilité aux différentes parties ressenties en touchant le membre remplacé. Une telle enveloppe est décrite dans les revendications 1-8.

Egalement décrit est un ensemble d'une enveloppe d'habillage, tel que mentionné ci-dessus, pour une prothèse de la jambe et d'une enveloppe d'habillage pour une prothèse de pied, selon les revendications 9-13.

Un procédé de fabrication d'une telle enveloppe selon l'invention est décrit dans les revendications 14 et 15.

### BRÈVE DESCRIPTION DES DESSINS

La présente invention sera mieux comprise sur la base de la description qui va suivre et des dessins en annexe sur lesquels :
[Fig. 1] montre un exemple d'une enveloppe d'habillage pour prothèse selon une première vue,
[Fig. 2] montre une anatomie d'un membre donné selon une première vue,
[Fig. 3] montre un exemple d'une enveloppe d'habillage pour prothèse selon une deuxième vue,
[Fig. 4] montre une anatomie d'un membre donné selon une deuxième vue,
[Fig. 5] montre une vue en coupe longitudinale d'une enveloppe d'habillage pour prothèse et une prothèse,
[Fig. 6] montre une vue en perspective d'un autre exemple d'enveloppe d'habillage pour prothèse et d'une enveloppe de pied prothétique,
[Fig. 7] montre un exemple d'une enveloppe de pied prothétique et d'un anneau comprenant des ergots,
[Fig. 8] montre une vue de détail de l'enveloppe d'habillage de prothèse de la figure 6 comprenant des ergots au niveau d'une extrémité de connexion à l'enveloppe de pied prothétique,
[Fig. 9] montre une vue agrandie de l'enveloppe d'habillage posée sur l'enveloppe de pied prothétique,
[Fig. 10] montre l'enveloppe d'habillage de la figure 9 séparée de l'enveloppe de pied prothétique.

### EXPOSÉ DÉTAILLÉ DE MODES DE RÉALISATION PARTICULIERS

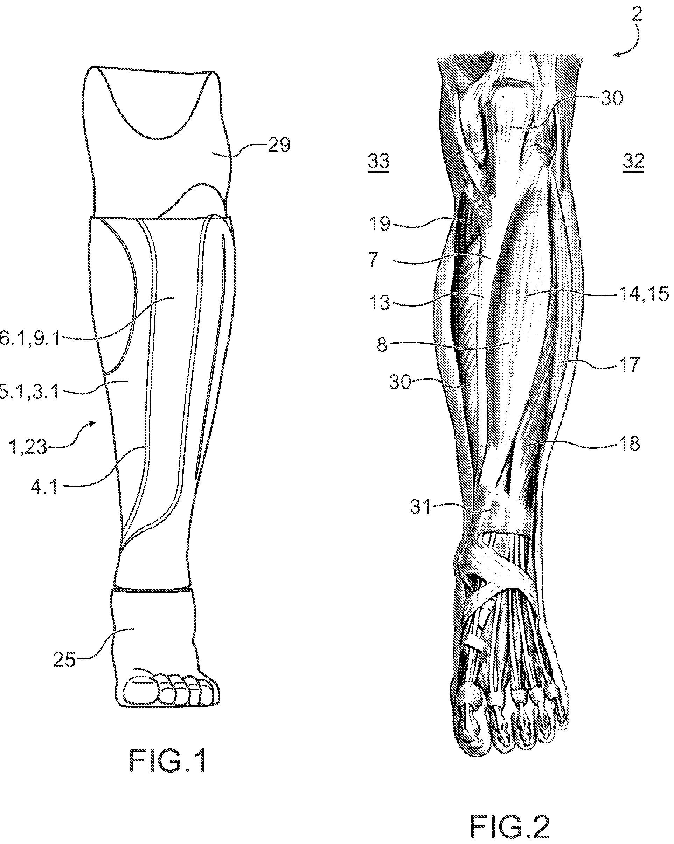

Un exemple de réalisation d'une enveloppe d'habillage pour prothèse est illustré en figures 1, 3, 5, 6 et 8 à 10.

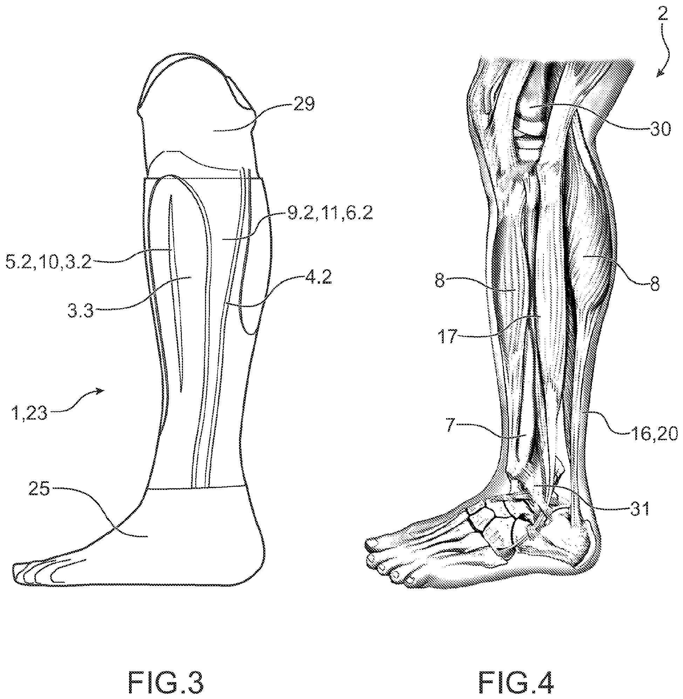

Les figures 2 et 4 montrent une anatomie d'un membre donné. Dans l'exemple présent, il s'agit de la partie inférieure d'une jambe. Plus précisément, il s'agit d'une partie tibiale d'une jambe humaine.

Sur lesdites figures on identifie des parties de la jambe ayant des duretés différentes (7, 8).

Entre le genou (30) et la cheville (31) s'étend le tibia (13), qui est l'un des os le plus important de ce membre inférieur. On observe également le tibial antérieur (14), qui est un des muscles (15) de la partie tibiale de la jambe humaine.

Le tibia (13) est situé sous une peau (non montré) de la jambe. Cet os se prolonge presque en ligne droite entre le genou et la cheville. Entre le tibia et la peau se situe le tibial antérieur (14). Ce muscle se prolonge, sous la peau, d'un côté extérieur (32) du genou jusqu'à un côté intérieur (33) de la cheville et traverse le tibia. On peut ainsi identifier une partie où le tibia se trouve directement sous la peau et une partie où le tibial antérieur se trouve directement sous la peau, entre la peau et le tibia.

En touchant la peau de la jambe on peut ainsi distinguer une première partie de dureté (7) et une deuxième partie de dureté (8), c'est à dire des parties du membre ayant des duretés différentes.

Au toucher, une partie sera ressentie comme molle si un muscle ou un tendon se trouve directement sous la peau. Une partie sera ressentie comme dure si un os se trouve directement sous la peau.

Dans le présent exemple, la partie de la jambe où le tibia se trouve directement sous la peau est une partie ressentie comme dure de, la partie de la jambe où le tibial antérieur se trouve directement sous la peau est une partie ressentie comme molle. On identifie ainsi deux parties de la jambe ayant des duretés différentes.

Touché de façon plus soigneuse, on peut différencier encore plus que deux parties de duretés différentes. Par exemple, une partie ayant plus de tissu musculaire entre la peau et le tibia sera plus dure qu'une partie ayant moins de tissu musculaire entre la peau et le tibia. Une partie où se trouve un tendon sera ressentie comme plus dure qu'une partie où se trouve un muscle, mais moins dure qu'une partie où se trouve un os.

D'autres parties molles et dures de la jambe sont formées par la position des autres os, muscles et tendons sur la jambe, comme le long fibulaire (17), le long extenseur des orteils (18), le gastrocnémien (19) ou le tendon d'Achille (20). On identifie ainsi une pluralité de parties de la jambe ayant des duretés différentes.

Des parties d'un membre ayant des duretés différentes ont été exemplifiées pour une jambe inférieure, mais elles peuvent également être retrouvées sur la partie supérieure de la jambe, sur le bras et à d'autres endroits du corps.

L'agencement des parties du membre ayant des duretés différentes, c'est à dire la délimitation d'une zone dure ou molle et la position relative d'une zone dure par rapport à une zone molle dépendent de l'anatomie du membre choisi. Cet agencement est une caractéristique du membre donné qui est remplacé par la prothèse.

Le présent exemple de réalisation porte sur une enveloppe pour une prothèse de la jambe, mais l'invention s'applique également à d'autres parties du corps présentant des duretés différentes.

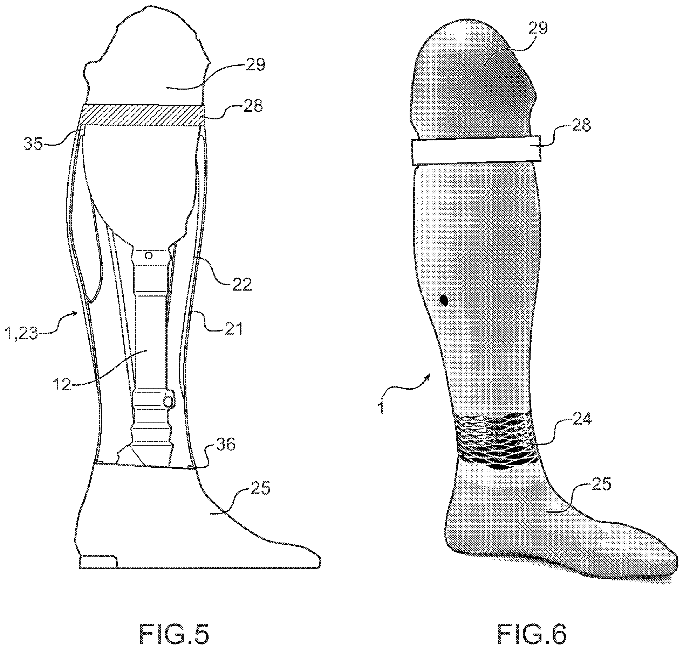

Les figures 1, 3 et 5 montrent une enveloppe d'habillage (1) pour une prothèse (12) de jambe. Sur les figures 1, 3 et 5 on identifie une enveloppe d'habillage ayant une forme tubulaire (23) et une enveloppe pour un pied prothétique (25). La prothèse se fixe par une emboîture (29) de prothèse sur un moignon d'un utilisateur (figure 5).

La figure 5 montre une surface intérieure (22) et une surface extérieure (21) de l'enveloppe. L'épaisseur de l'enveloppe est une quantité de matériau compris entre ladite surface intérieure et surface extérieure de l'enveloppe. On identifie sur les figures 1 et 3 également une première épaisseur (3.1, 3.2, 3.3) de l'enveloppe, une deuxième épaisseur (9.1, 9.2) et un changement d'épaisseur (4.1, 4.2) situé entre la première (3.1, 3.2, 3.3) et la deuxième (9.1, 9.2) épaisseur.

Par forme tubulaire on comprend une forme cylindrique qui peut présenter des coupes ayant une forme et une taille variable. La forme tubulaire peut également être courbée et ainsi suivre majoritairement un axe non droit. L'enveloppe montrée dans la figure 5 présente ainsi une coupe de forme et de taille qui est différent à un niveau du mollet et à un niveau de la cheville. Cette enveloppe de forme tubulaire pourrait également être courbée et ainsi suivre une emboîture (29) qui présente un angle par rapport à la prothèse (12). Une enveloppe qui a une forme de jambe ou de bras présente ainsi une forme tubulaire.

Le changement d'épaisseur délimite ainsi une première zone (5.1, 5.2) ayant la première épaisseur (3.1, 3.2, 3.3) d'une deuxième zone (6.1, 6.2) ayant la deuxième épaisseur (9.1, 9.2).

L'enveloppe présente une flexibilité en dépendance de son épaisseur. Ainsi, si la première zone (5.1, 5.2) a une épaisseur plus importante que la deuxième zone (6.1, 6.2), la première zone (5.1, 5.2) aura une flexibilité moins importante que la deuxième zone (6.1, 6.2).

Autrement dit, si la première zone (5.1, 5.2) à une épaisseur plus importante qu'une épaisseur moyenne de l'enveloppe et si la deuxième zone (6.1, 6.2) a une épaisseur moins importante qu'une épaisseur moyenne de l'enveloppe, la première zone (5.1, 5.2) aura une flexibilité moins importante que la deuxième zone (6.1, 6.2).

Des zones de flexibilité différentes peuvent aussi être obtenues par une différence de matériau. Une première zone peut être fabriquée d'un premier matériau, une deuxième zone peut être fabriquée d'un deuxième matériau. Le premier matériau peut être moins flexible que le deuxième matériau. On obtient ainsi deux zones de flexibilité différentes.

Une différence de matériau peut être comprise comme une différence de composition chimique du matériau. Ainsi, un polymère utilisé peut être plus flexible s'il comprend une composante chimique ajoutée, par exemple s'il comprend un plastifiant.

Une différence de matériau peut également être compris comme une différence en composition macroscopique. Ainsi, une deuxième zone peut comprendre un polyuréthane thermoplastique. Une première zone peut comprendre le polyuréthane thermoplastique et aussi des fibres de carbone. On obtient ainsi deux zones de flexibilité différentes, la première zone ayant une flexibilité moins importante que la deuxième zone à cause des propriétés des fibres de carbone.

Il est également possible d'obtenir des zones de flexibilité différentes par une différence de motif en trois dimensions imprégné sur la zone. Par exemple, une deuxième zone peut comprendre un polyuréthane thermoplastique. Une première zone peut comprendre l'uréthane thermoplastique imprégné d'une déformation en trois dimensions rendant ladite zone moins flexible.

En touchant l'enveloppe, la zone d'une flexibilité plus importante donne l'impression de toucher une partie molle d'un membre alors que la zone de flexibilité moins importante donne l'impression de toucher une partie dure d'un membre.

L'enveloppe d'habillage peut aussi comprendre une pluralité de zones présentant des flexibilités différentes. On peut prévoir deux, trois, quatre ou plus de zones, l'ensemble des zones présentant deux, trois ou plus de flexibilités différentes. Par exemple, on peut prévoir cinq zones, l'ensemble des cinq zones ayant trois flexibilités différentes.

De cette façon, il est possible d'obtenir une pluralité de flexibilités différentes. On peut ainsi obtenir une enveloppe d'habillage qui fait sentir, au toucher, une pluralité de duretés différentes. De cette façon, l'enveloppe d'habillage peut encore plus précisément ressembler à un toucher d'une anatomie d'un membre parce que, touché de façon plus soigneuse, on peut différencier plus de deux parties de duretés différentes sur l'anatomie d'un membre. Il est également possible de varier une combinaison d'une épaisseur, d'un matériau et/ou d'une structure en trois dimensions pour obtenir des zones de flexibilité différentes.

De différentes épaisseurs de l'enveloppe peuvent être fabriquées en faisant varier une épaisseur d'un matériau utilisé pour une fabrication de l'enveloppe. Il est également possible d'utiliser un matériau comprenant plusieurs couches et de varier d'un endroit à un autre endroit le nombre de couches pour arriver à l'épaisseur différente de l'enveloppe. Il est également possible d'utiliser des couches faites de différents matériaux.

En comparant l'enveloppe de la figure 1 avec l'anatomie de la figure 2 on observe que l'agencement des zones de flexibilité différentes (5.1, 5.2, 6.1, 6.2) entre elles correspond à l'agencement des parties du membre ayant des duretés différentes (13, 14, 17).

On observe sur la figure 1 que la deuxième zone (6.1) est agencée de façon majoritairement longitudinale en se courbant vers le bas. La première zone (5.1) est agencée de façon à s'élargir d'un haut vers un bas.

La première zone (5.1) est ainsi agencée par rapport à la deuxième zone (6.1) sur l'enveloppe comme le tibia (13) est agencé par rapport au tibial antérieur (14).

L'agencement des zones varient d'un patient à un autre, comme la position des os, muscles et tendons varie d'un patient à un autre.

Dans l'exemple présent, la première zone (5.1) a une épaisseur plus importante que la deuxième zone (6.1). Ladite première zone donne ainsi au toucher l'impression d'être plus dure que la deuxième zone. La deuxième zone donne l'impression d'être plus molle que la première zone.

En touchant l'enveloppe, l'utilisateur reçoit ainsi une sensation similaire à toucher une partie inférieure d'une jambe humaine. La première zone (5.1) sera ressentie comme en touchant le tibia et la deuxième zone comme en touchant le muscle tibial antérieur. En glissant un doigt entre la première zone (5.1) et la deuxième zone (6.1), le changement de flexibilité, provoqué par le changement d'épaisseur de l'enveloppe, est ressenti comme un changement de fermeté, entre une partie molle et une partie dure d'une jambe humaine.

De la même façon, on observe sur les figures 3 et 4 qu'une deuxième zone (6.2) ayant une épaisseur moins importante est agencée sur l'enveloppe comme le long fibulaire (17) sur la partie tibiale d'une jambe.

Un changement d'épaisseur pour délimiter des zones de flexibilités différentes peut aussi être obtenu par une nervure ou par une échancrure sur l'enveloppe d'habillage.

La figure 3 montre une nervure (10) sur l'enveloppe d'habillage pour obtenir une flexibilité moins importante à l'endroit où se trouve la nervure. La figure 3 montre également une échancrure (11) pour obtenir une flexibilité augmentée à l'endroit où se trouve l'échancrure.

Une nervure où une échancrure présente un changement d'épaisseur de l'enveloppe. Une enveloppe ayant une nervure comprend une première zone et une deuxième zone ayant des épaisseurs différentes. On peut, par exemple, identifier comme première zone d'épaisseur plus importante la zone de la nervure elle-même. La deuxième zone d'épaisseur moins importante par rapport à la première zone est, dans ce cas, la zone qui se trouve en dehors de l'emplacement de la nervure.

Une utilisation de nervures ou d'échancrures permet un agencement particulièrement fin de zones de flexibilité différentes.

De façon avantageuse, l'enveloppe comprend un polyuréthane thermoplastique (thermoplastic polyurethane, TPU) et/ou un polyamide.

Le polyuréthane thermoplastique présente des avantages pour la fabrication d'une enveloppe de prothèse. Il permet une bonne imitation d'un membre controlatéral, ce matériau peut aller dans l'eau et peut être lavé facilement. Sous un choc, l'enveloppe comprenant ce matériau se déforme et ne fait pas de bruit. Du fait de ses propriétés d'élasticité et de flexibilité, le TPU est particulièrement bien adapté pour la fabrication de zones de flexibilité différentes pour imiter un toucher physiologique. Une enveloppe comprenant ce matériau est léger et ne rajoute pas trop de poids à la prothèse.

L'enveloppe peut être fabriquée par un procédé d'impression 3D, par exemple par fusion de poudre.

La première zone (5.1, 5.2) et la deuxième zone (6.1, 6.2) peuvent avoir une forme longitudinale (figure 1, 2) sur l'enveloppe d'habillage, la forme longitudinale se prolongeant le long d'une extension du membre.

Dans l'exemple de réalisation montré aux figures 1, 3 et 5, l'enveloppe d'habillage a une forme tubulaire. La première épaisseur (3.1, 3.2, 3.3) et la deuxième épaisseur (9.1, 9.2) restent majoritairement constantes le long d'une longueur de l'enveloppe. L'épaisseur présente un changement majoritairement le long d'une circonférence de la forme tubulaire de l'enveloppe.

Dans le cas particulier de nervures ou d'échancrures, lesdites nervures et échancrures se prolongent majoritairement le long d'une longueur de la forme tubulaire.

Un agencement tel que décrit ci-dessus correspond particulièrement bien pour la fabrication d'une enveloppe de prothèse pour un bras ou pour une jambe.

Les muscles, tendons et les os du bras et de la jambe sont allongés majoritairement le long d'une longueur du bras ou de la jambe. Pour cette raison, en touchant un bras ou une jambe, on ressent peu de changements de dureté en se promenant le long du membre. Une nervure ou une échancrure prévue le long d'une longueur de la forme tubulaire provoque un changement de dureté ressenti si un utilisateur promène son doigt sur la circonférence de l'enveloppe, la dureté restant constant le long de la longueur de la forme tubulaire. Au toucher, l'enveloppe reproduit ainsi un comportement similaire à une jambe ou à un bras.

La figure 5 montre l'enveloppe d'habillage (1) pour une prothèse de tibia et une enveloppe pour pied prothétique (25). Une prothèse de membre inférieur (12), plus particulièrement une prothèse de tibia, comprenant une emboîture de prothèse (29) est également montrée. Dans l'exemple montré, la liaison entre l'emboîture et la prothèse de pied est assuré par un élément intérieur (12), par exemple une tige ou une barre.

De la même façon (image non montrée), on peut prévoir une cage rigide pour assurer une liaison entre l'emboîture et la prothèse de pied. Dans ce cas, la cage remplace le tube mentionné ci-dessus. La cage assure la stabilité et la transmission d'une force entre la prothèse de pied et l'emboîture. Dans ce cas, l'enveloppe d'habillage loge la cage en son intérieur et enveloppe ainsi la cage.

L'utilisation d'une cage est particulièrement bien adaptée pour la mise en œuvre d'une prothèse de bras. La cage assure la liaison entre l'emboîture et une prothèse de la main. La prothèse elle-même se présente sous forme de cage en matériau rigide, ladite cage étant fixée par une extrémité sur une emboîture de prothèse. La prothèse de la main est fixée sur l'autre extrémité de la cage. L'enveloppe d'habillage est fixée sur le cage. Une enveloppe de la main prothétique peut être fixée sur la main prothétique. Le cage peut être fabriqué en fibre de carbone ou en polyamide thermoplastique, par exemple en PA12. L'enveloppe d'habillage pour la prothèse du bras et l'enveloppe d'habillage pour la prothèse de la main peuvent être fabriquées en deux parties. Elles peuvent aussi être fabriquées venu de matière l'une avec l'autre et de cette façon couvrir en un seul élément de matière continue la prothèse du bras et la prothèse de la main.

L'enveloppe a une forme tubulaire (3) comprenant une surface intérieure (22) et une surface extérieure (21). L'enveloppe loge la prothèse en son intérieur et enveloppe ainsi la prothèse. L'enveloppe d'habillage est posée avec son bord inférieur (36) sur l'enveloppe pour pied prothétique (25).

De façon avantageuse, la surface extérieure (21) de l'enveloppe est majoritairement lisse par rapport aux changements (4.1, 4.2) délimitant des zones de flexibilité différentes.

La dilatation ou la rétractation du matériau qui forme une épaisseur plus importante (par exemple une nervure) ou moins importante (par exemple une échancrure) a lieu vers un intérieur de l'enveloppe (figure 5), en direction de la prothèse. La surface reste, de cette façon, lisse.

Plus précisément, la surface intérieure (22) s'éloigne ou se rapproche de la surface extérieure (21) pour provoquer un changement de l'épaisseur. L'ensemble de la surface extérieure et de la surface intérieure, formant une paroi de l'enveloppe, peut épouser une forme ressemblant à une anatomie d'un membre. On observe, par exemple sur la figure 5, que la paroi entre la surface extérieure (21) et la surface intérieure (22) se courbe d'un haut vers un bas pour faire ressembler l'enveloppe, à cet endroit, à un mollet. Une surface extérieure lisse signifie que la surface intérieure, tout en suivant la forme de la surface extérieure, s'éloigne et se rapproche de la surface extérieure pour former des zones d'épaisseur différentes. Dans le cas particulier de nervures (10) et/ou d'échancrures (11), majoritairement lisse signifie que la nervure et/ou l'échancrure est situé à l'intérieur de l'enveloppe.

L'enveloppe confère par son extérieur lisse encore plus un toucher physiologique, correspondant à une peau lisse ayant des duretés différentes au toucher.

Il est également possible de prévoir une surface intérieure majoritairement lisse et de situer les changements (4.1, 4.2) sur une surface extérieure. Ainsi, les nervures et/ou les échancrures peuvent être orientés vers une surface extérieure.

Aussi, les changements (4.1, 4.2) peuvent être situés sur la surface extérieure et sur la surface intérieure.

La figure 6 montre l'enveloppe d'habillage (1) pour une prothèse de la jambe, l'enveloppe ayant une forme tubulaire et comprenant une zone d'articulation (24). On trouve également l'enveloppe pour pied prothétique (25) qui peut être fabriquée en mousse de polyuréthane.

L'enveloppe d'habillage (1) se pose sur l'enveloppe pour pied prothétique. A l'intérieur de l'enveloppe d'habillage est positionnée la prothèse (12) montré également sur la figure 5.

Dans l'exemple exposé, la zone d'articulation (24) est prévue au niveau d'une cheville, mais elle pourrait se trouver également à un niveau de genou si l'enveloppe est prévue pour un autre type de prothèse, comme une prothèse pour une jambe entière.

Dans le présent exemple, la zone d'articulation (24) comporte des ouvertures dans l'enveloppe d'habillage. Plus précisément, il s'agit dans le présent exemple d'ouvertures ayant une forme de losange, situées à un niveau de la cheville. D'autres formes d'ouvertures peuvent être utilisées, par exemple sous forme de cercle ou de rectangle. Du fait du matériel manquant dans les ouvertures, l'enveloppe est plus flexible à l'endroit des ouvertures qu'ailleurs. L'enveloppe peut ainsi se déformer facilement au niveau de la cheville pour suivre le mouvement d'une prothèse durant une marche.

Les ouvertures en forme de losange permettent également d'accéder à la prothèse située à l'intérieur de l'enveloppe d'habillage. Dans l'exemple ci-présent (figure 6), il est possible d'accéder à la prothèse à travers des ouvertures en forme de losange. On peut ainsi déconnecter le pied de la prothèse des autres parties de la prothèse sans avoir à modifier l'enveloppe de la prothèse. Egalement, des procédures de maintenance sont possibles sans la nécessité d'enlever l'enveloppe d'habillage. D'autres ouvertures, par exemple à un niveau du mollet, au-dessus de la zone d'articulation (24) (voir figure 6), peuvent être prévues pour permettre d'accéder à d'autres éléments de réglage de la prothèse (par exemple une valve, un détrompeur). La répartition de ces ouvertures est propre à chaque prothèse et dépendant des besoins de chaque patient.

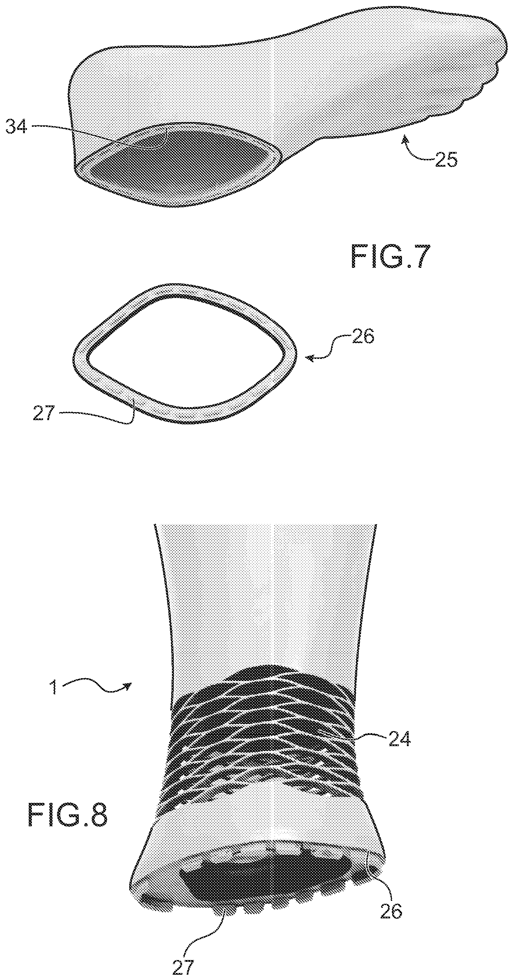

Les figures 7 à 10 montrent l'enveloppe d'habillage (1) ayant la zone d'articulation (24) et l'enveloppe pour pied prothétique (25). L'enveloppe pour pied présente des évidements (34) sur un côté ouvert.

La figure 8 montre également des ergots (27) sur un bord inférieur de l'enveloppe (36), mais dans certains cas un seul ergot pourrait suffire pour assurer une connexion stable.

Selon un premier mode de réalisation, l'ergot (27) et l'enveloppe d'habillage (1) sont formés en une seule pièce. Les ergots sont venus de matière avec l'enveloppe d'habillage.

La figure 7 montre un anneau (26) ayant une première surface et une deuxième surface. La deuxième surface est munie d'ergots (27). Selon un deuxième mode de réalisation, la première surface de l'anneau (26) est fixée, de préférence collée, sur le bord inférieur de l'enveloppe de prothèse.

L'enveloppe d'habillage comprend ainsi au moins un ergot (27) sur un bord inférieur (36).

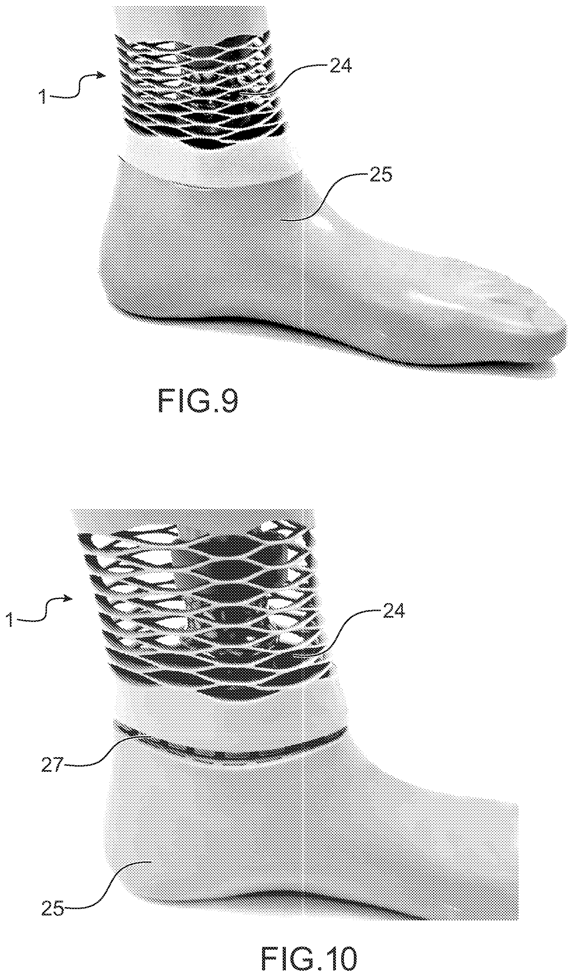

Ce bord inférieur se trouvera face à l'enveloppe pour pied prothétique (figure 9, 10).

Les ergots (27) sont de cette façon orientés vers l'extérieur, en direction de l'enveloppe pour pied (figure 10). L'enveloppe d'habillage (1) peut se fixer sur l'enveloppe pour pied (25) pour former un habillage complet d'une prothèse. Au montage, les ergots de l'anneau s'emboîtent de manière réversible dans les trous (34) prévus sur le côté ouvert de l'enveloppe pour pied prothétique (figure 9, 10). Le montage étant réversible, l'enveloppe d'habillage peut être séparée de l'enveloppe pour pied en tirant légèrement sur l'une des deux pièces. La prothèse tibiale se démonte ainsi aisément sans nécessité de modifier, comme en coupant ou en déformant, l'enveloppe d'habillage.

A travers des ouvertures de la zone d'articulation on accède, si nécessaire avec un outil, à la prothèse située à l'intérieur de l'enveloppe d'habillage. D'autres ouvertures peuvent être présentes pour accéder à des éléments de réglage différents de la prothèse, comme une valve ou un détrompeur.

On arrive ainsi à démonter le pied prothétique du restant de la prothèse. Ensuite, on enlève le pied prothétique du restant de la prothèse en séparant l'enveloppe d'habillage de l'enveloppe pour pied prothétique en déboîtant les ergots des trous (34).

Selon un autre mode de réalisation, l'enveloppe d'habillage peut être venue de matière avec l'enveloppe du pied prothétique. Selon ce mode de réalisation, l'enveloppe d'habillage forme un élément de matière continue avec le pied prothétique.

Les figures 5 et 6 montrent également un collet de serrage (28) sur une partie haute (35) de l'enveloppe d'habillage.

Le collet de serrage fixe l'enveloppe (1) sur l'emboîture de prothèse (29). Le collet est agencé de façon à permettre un léger glissement entre l'enveloppe et l'emboîture afin que l'esthétique ne soit pas en contrainte lors d'une marche avec la prothèse comprenant l'enveloppe.

L'enveloppe décrite ci-dessus peut être fabriqué avantageusement par le procédé décrit par la suite.

Dans une première étape, une image en trois dimensions d'un membre est obtenue.

Par exemple, un membre non-amputé du patient est numérisé en utilisant un scanner 3D pour obtenir ladite image. Par ladite image on obtient une représentation de la surface de ce membre, c'est à dire on obtient une représentation numérique en trois dimensions du membre. La représentation numérique du membre est une surface en trois dimensions.

Alternativement, une image en trois dimensions sortie d'une bibliothèque de données pourrait-être utilisée. Dans ce cas, ladite image est obtenue à partir d'une bibliothèque de morphotypes basés sur la taille et le poids du patient.

On pourrait aussi se servir d'une image scannée du membre du patient faite avant l'amputation du membre.

Dans l'exemple présent on obtient une représentation numérique d'une partie tibiale d'une jambe, c'est à dire une représentation numérique d'une partie inférieure de la jambe tel que montrée aux figures 2 et 4.

De la même façon, on peut également obtenir une représentation numérique d'un pied. Comme décrit ci-dessus, il est possible d'utiliser un scanner 3D ou de récupérer une image d'une bibliothèque d'images.

De la même façon, on peut également obtenir une représentation numérique d'une jambe avec son pied, en utilisant un scanner 3D ou en récupérant une image d'une bibliothèque d'images.

De la même façon, on peut également obtenir une représentation numérique d'une main, d'un bras avec sa main ou d'un bras et d'une main séparément.

Dans une deuxième étape, les parties du membre ayant des duretés différentes (7, 8) sont identifiées sur la représentation numérique du membre. Cette identification peut se faire de façon automatique, de façon manuelle par un opérateur ou par un opérateur assisté par un algorithme. Cette identification peut se faire sur la représentation numérique complète ou seulement sur une partie de la représentation numérique. Dans l'exemple d'une représentation numérique d'une jambe avec son pied, cette identification peut se faire, par exemple, seulement sur la partie de la jambe en excluant le pied. A la suite de cette étape, une délimitation des parties ayant des duretés différentes peut être tracée sur la représentation du membre. Autrement dit un agencement des parties de dureté différentes entre elles peut être tracée sur la représentation du membre.

Par exemple, sur une représentation numérique d'une partie tibiale d'une jambe, on identifie la partie correspondant au tibia et la partie correspondant au tibial antérieur. Ensuite, on trace sur la représentation numérique une ligne pour délimiter une extension du tibia et du tibial antérieur sous la peau.

Dans une troisième étape, l'esthétique est conçue. La deuxième et la troisième étape peuvent être réalisées en utilisant un logiciel de conception assistée par ordinateur (CAO).

Sur un modèle numérique de l'enveloppe, une zone est définie en définissant sa forme. On trace alors sur la surface de l'enveloppe une forme de zone. Ensuite, une flexibilité de cette zone est définie. On fixe par exemple l'épaisseur que le matériau de l'enveloppe va avoir au sein de cette zone. A la fin de cette étape, un agencement de zones de différentes flexibilités, par exemple obtenu par différentes épaisseurs, est défini sur l'enveloppe.

La forme et la flexibilité des zones sont définies de façon à correspondre à l'agencement des parties du membre ayant des duretés différentes.

Dans un exemple utilisant des épaisseurs différentes, sur l'enveloppe, une première zone ayant une épaisseur plus importante sera située par rapport à une deuxième zone ayant une épaisseur moins importante comme une partie du membre plus dure se situe par rapport à une partie moins dure sur la représentation numérique du membre, lesdites parties étant identifiées auparavant comme décrit ci-dessus.

Pour vérifier l'agencement desdites zones entre elles et la correspondance à l'agencement des parties du membre on peut projeter la représentation numérique de l'enveloppe pour prothèse sur la représentation numérique du membre.

De cette façon, une zone ayant une épaisseur plus importante qu'une épaisseur moyenne se trouve arrangée pour coïncider avec une partie dure du membre et/ou une zone ayant une épaisseur moins importante qu'une épaisseur moyenne se trouve arrangée pour coïncider avec une partie molle du membre.

Par exemple, on trace sur un modèle numérique de l'enveloppe tel qu'on le voit à la figure 1 une première zone (5.1) correspondant par sa forme à la partie (13) du tibia. La partie du tibia a été auparavant identifiée sur la représentation numérique de la jambe.

Ensuite, on trace une deuxième zone (6.1) sur le modèle numérique de l'enveloppe correspondant par sa forme à la partie (14) du tibial antérieur, tel que identifiée sur la représentation numérique de la jambe auparavant.

Ensuite, on associe une épaisseur plus importante avec la première zone et une épaisseur moins importante avec la deuxième zone.

L'enveloppe fabriquée sera ainsi moins flexible au niveau de la première zone qu'au niveau de la deuxième zone, tel qu'une jambe est plus dure à une partie correspondant au tibia et plus molle à une partie correspondant tibial antérieur. L'enveloppe confère de cette façon un toucher physiologique à un utilisateur.

Il est également possible de donner à l'enveloppe la forme du membre tel qu'elle est déduite de la représentation numérique du membre d'origine. L'enveloppe adopte de cette façon la forme du membre. L'enveloppe peut ainsi adopter la forme en trois dimensions de la jambe, par exemple de la partie tibiale, du bras, du pied ou de la main. L'enveloppe peut également adopter la forme de la jambe avec son pied ou du bras avec sa main et ainsi reproduire une forme complète d'un membre.

Une enveloppe qui adopte la forme du membre et confère en même temps un toucher physiologique est particulièrement bien adapté pour fournir à un utilisateur un sentiment naturel quand il porte sa prothèse.

Le procédé de fabrication peut également comprendre une étape de numérisation en 3D de la prothèse (12) qu'on veut couvrir par l'enveloppe, pour adapter l'enveloppe à la prothèse durant le procédé de conception assistée par ordinateur.

L'enveloppe peut ensuite être fabriquée par un procédé d'impression 3D à partir du modèle numérique. Plus particulièrement, on peut utiliser une impression en 3D par fusion de poudre, par exemple en TPU.

Il est ainsi possible de fabriquer une enveloppe d'habillage pour une prothèse de la jambe ou pour une prothèse du bras.

Dans le cas d'une prothèse de la jambe, l'enveloppe d'habillage peut être fabriquée à partir d'une représentation numérique d'une jambe complète avec son pied, par le procédé décrit ci-dessus. Dans ce cas, il est possible de fabriquer un habillage complet pour la prothèse du pied et pour la prothèse de la jambe. L'habillage pour la prothèse de la jambe et l'habillage pour la prothèse du pied peuvent être fabriquées en deux parties séparées. L'habillage pour la prothèse de la jambe et l'habillage pour la prothèse du pied peuvent aussi être fabriqués venu de matière l'une avec l'autre. On obtient dans le dernier cas un habillage de prothèse qui peut couvrir en un seul élément de matière continue la prothèse du pied et la prothèse de la jambe. En d'autres mots, à partir d'une représentation numérique d'une jambe complète avec son pied on peut fabriquer une enveloppe couvrant la prothèse de la jambe et la prothèse du pied par un seul élément de matière continue. On peut conférer à cet habillage la forme tel que définie par la représentation numérique. En d'autres mots, l'habillage de la prothèse de la jambe reproduit la forme de la jambe selon la représentation numérique et l'habillage de la prothèse du pied reproduit la forme du pied selon la représentation numérique. En résumé, on peut obtenir à partir d'une représentation d'une jambe complète avec son pied un habillage d'une prothèse complète, incluant une prothèse tibiale avec une prothèse de pied. Cet habillage peut avoir la forme de la jambe complète avec son pied et conférer, par endroits ou complètement, un toucher physiologique.

De la même façon on peut obtenir un habillage pour une prothèse de membre supérieur, par exemple d'un bras. Dans le cas d'une prothèse du bras, l'habillage peut également être fabriqué à partir d'une représentation numérique d'un bras complet avec sa main, par le procédé décrit ci-dessus. L'habillage pour la prothèse du bras et l'habillage pour la prothèse de la main peuvent être fabriqués en deux parties séparées ou venues de matière l'une avec l'autre. En d'autres mots, il est possible de fabriquer à partir d'une représentation numérique du bras complet avec sa main une enveloppe couvrant la prothèse du bras et la prothèse de la main par un seul élément de matière continue. L'habillage peut adopter la forme telle que définie par la représentation numérique du bras avec ou sans sa main.

## Revendications

1. Enveloppe d'habillage (1) pour prothèse (12) d'un membre (2) donné, l'enveloppe comprenant au moins deux zones (5.1, 5.2, 6.1, 6.2) de flexibilité différentes (3.1, 3.2, 3.3, 9.1, 9.2), **caractérisé en ce que**
l'agencement desdites zones entre elles correspondant à l'agencement de parties du membre donné ayant des duretés différentes (7, 8).

2. Enveloppe selon la revendication 1, dans laquelle une première zone (5.1, 5.2) diffère d'une deuxième zone (6.1, 6.2) par rapport à :
- une épaisseur et/ou
- un matériau et/ou
- un motif en trois dimensions
afin d'obtenir une première zone ayant une flexibilité différente d'une deuxième zone.

3. Enveloppe selon la revendication 1 ou 2, dans laquelle :
- une première zone a une flexibilité moins (3.1, 3.2, 3.3) importante qu'une deuxième zone, la première zone étant arrangée pour coïncider avec une partie dure (7) du membre, et/ou
- une deuxième zone a une flexibilité plus importante (9.1, 9.2) que une première zone, la deuxième zone étant arrangée pour coïncider avec une partie molle (8) du membre, de préférence
dans laquelle :
- la première zone (5.1, 5.2) et la deuxième zone (6.1, 6.2) présentent des épaisseurs différentes, et
- la première zone et la deuxième zone sont une nervure (10) ou une échancrure (11).

4. Enveloppe selon l'une des revendications 1 à 3, dans laquelle l'enveloppe a une forme tubulaire apte à recevoir ladite prothèse (12) à l'intérieur de la forme tubulaire, de préférence dans laquelle :
- une flexibilité de l'enveloppe reste majoritairement constante le long d'une longueur de la forme tubulaire, et/ou
- la flexibilité de l'enveloppe change majoritairement le long d'une circonférence de la forme tubulaire.

5. Enveloppe selon l'une des revendications précédentes, l'enveloppe étant adaptée pour loger en son intérieur une prothèse de jambe, de préférence une prothèse de tibia ou une prothèse de bras.

6. Enveloppe selon les revendications 2 et 5 dans laquelle :
- une zone ayant une épaisseur plus importante (3.1, 3.2, 3.3) qu'une épaisseur moyenne de l'enveloppe est située sur l'enveloppe comme le tibia (13) est situé sous une peau d'une jambe et/ou
- une zone ayant une épaisseur moins importante (6.1, 6.2) qu'une épaisseur moyenne de l'enveloppe est située sur l'enveloppe comme un muscle (15) ou un tendon (16) est situé sous une peau d'une jambe,
le muscle ou le tendon étant de préférence le soléaire (30), le tibial antérieur (14), le long fibulaire (17), le long extenseur des orteils (18), le gastrocnémien (19) ou le tendon d'Achille (20).

7. Enveloppe selon l'une des revendications précédentes en combinaison avec la revendication 2, dans laquelle une surface extérieure (21) de l'enveloppe est majoritairement lisse et les changements d'épaisseur sont situés à une surface intérieure (22) de l'enveloppe.

8. Enveloppe selon l'une des revendications précédentes, dans laquelle :
- un matériau de l'enveloppe comprend un polyuréthane thermoplastique (TPU) et/ou
- un matériau de l'enveloppe comprend un polyamide.

9. Ensemble d'une enveloppe d'habillage pour une prothèse de la jambe et d'une enveloppe d'habillage pour une prothèse de pied,
l'enveloppe d'habillage étant selon l'une des revendications 1 à 8,
l'enveloppe d'habillage de la prothèse de la jambe étant venue de matière avec l'enveloppe d'habillage de la prothèse du pied.

10. Ensemble d'une enveloppe d'habillage pour prothèse selon l'une des revendications 1 à 8 et d'une enveloppe pour un pied prothétique (25), l'enveloppe d'habillage ayant une forme tubulaire (23) et comprenant au moins un ergot (27) sur un bord inférieur (36) de la forme tubulaire, l'ergot étant apte à s'emboîter de manière réversible dans l'enveloppe du pied prothétique (25), de façon à le fixer de façon démontable à la forme tubulaire, de préférence comprenant un anneau (26) avec une première surface et une deuxième surface, la première surface de l'anneau comprenant l'ergot (27), la deuxième surface de l'anneau étant fixée, de préférence collée, sur le bord inférieur (36) de la forme tubulaire.

11. Enveloppe selon l'une des revendications 1 à 8
dans laquelle l'enveloppe comprend un collet de serrage (28), ledit collet étant apte à fixer une partie supérieure de la forme tubulaire sur une emboîture (29) de prothèse de façon à permettre un glissement entre la forme tubulaire et l'emboîture.

12. Ensemble selon l'une des revendications 9 ou 10,
dans lequel l'enveloppe comprend un collet de serrage (28), ledit collet étant apte à fixer une partie supérieure de la forme tubulaire sur une emboîture (29) de prothèse de façon à permettre un glissement entre la forme tubulaire et l'emboîture.

13. Ensemble d'une enveloppe d'habillage pour une prothèse de bras et d'une enveloppe d'habillage pour une prothèse de la main,
l'enveloppe d'habillage étant selon l'une des revendications 1 à 8,
l'enveloppe d'habillage de la prothèse du bras étant venue de matière avec l'enveloppe d'habillage de la prothèse de la main ou
l'enveloppe d'habillage de la prothèse de la main se fixant de façon démontable à l'enveloppe d'habillage de la prothèse du bras.

14. Procédé de fabrication d'une enveloppe selon l'une des revendications 1 à 13, le procédé comprenant les étapes suivantes :
- obtenir une image en trois dimensions d'un membre,
- identifier sur l'image les parties du membre ayant des duretés différentes (7, 8),
- définir sur un modèle numérique de l'enveloppe des zones de flexibilité différentes (5.1, 5.2, 6.1, 6.2) de façon à correspondre à l'agencement desdites parties identifiées sur ladite image,
- réalisation de l'enveloppe à partir du modèle numérique, de préférence dans lequel
- l'image en trois dimensions est obtenue à partir d'une jambe complète avec son pied ou à partir d'un bras complet avec sa main,
- l'enveloppe comprend l'enveloppe de la prothèse de la jambe et l'enveloppe de la prothèse du pied ou l'enveloppe de la prothèse du bras et l'enveloppe de la prothèse de la main, de préférence le procédé comprenant l'étape suivante :
- identifier sur l'image la forme du membre
- définir le modèle numérique de l'enveloppe de façon à ce que la forme de l'enveloppe corresponde à la forme du membre telle qu'identifiée sur l'image.

15. Procédé selon la revendication 14, dans lequel l'étape de réalisation est une étape d'impression en 3D, de préférence par fusion de poudre, de l'enveloppe à partir dudit modèle numérique.

## Patentansprüche

1. Verkleidungshülle (1) für eine Prothese (12) eines bestimmten Gliedmaßes (2), wobei die Hülle mindestens zwei Bereiche (5.1, 5.2, 6.1, 6.2) unterschiedlicher Flexibilität (3.1, 3.2, 3.3, 9.1, 9.2) umfasst, **dadurch gekennzeichnet, dass**
die Anordnung der Bereiche untereinander der Anordnung von Teilen der jeweiligen Gliedmaße mit unterschiedlichen Härten (7, 8) entspricht.

2. Hülle nach Anspruch 1, wobei sich ein erster Bereich (5.1, 5.2) von einem zweiten Bereich (6.1, 6.2) in Bezug auf Folgendes unterscheidet:
- eine Dicke und/oder
- ein Material und/oder
- ein dreidimensionales Muster
- ein dreidimensionales Muster, um einen ersten Bereich mit einer anderen Flexibilität als einen zweiten Bereich zu erhalten.

3. Hülle nach Anspruch 1 oder 2, wobei:
- ein erster Bereich eine geringere Flexibilität (3.1, 3.2, 3.3) als ein zweiter Bereich aufweist, wobei der erste Bereich so angeordnet ist, dass er mit einem harten Teil (7) der Gliedmaße zusammenfällt, und/oder
- ein zweiter Bereich eine größere Flexibilität (9.1, 9.2) als ein erster Bereich aufweist, wobei der zweite Bereich so angeordnet ist, dass er mit einem weichen Teil (8) der Gliedmaße zusammenfällt, wobei
vorzugsweise:
- ein erster Bereich (5.1, 5.2) und ein zweiter Bereich (6.1, 6.2) unterschiedliche Dicke besitzen, und
- der erste Bereich und der zweite Bereich eine Rippe (10) oder eine Aussparung (11) sind.

4. Hülle nach einem der Ansprüche 1 bis 3, wobei die Hülle eine röhrenförmige Form aufweist, die geeignet ist, die Prothese (12) innerhalb der röhrenförmigen Form aufzunehmen, wobei vorzugsweise:
- eine Flexibilität der Hülle entlang einer Länge der röhrenförmigen Form überwiegend konstant bleibt, und/oder
- die Flexibilität der Hülle sich entlang eines Umfangs der röhrenförmigen Form überwiegend ändert.

5. Hülle nach einem der vorhergehenden Ansprüche, wobei die Hülle geeignet ist, in ihr eine Beinprothese, vorzugsweise eine Tibiaprothese oder eine Armprothese aufzunehmen.

6. Hülle nach Anspruch 2 und 5, wobei
- ein Bereich mit einer größeren Dicke (3.1, 3.2, 3.3) als eine durchschnittliche Dicke der Hülle auf der Hülle liegt, da das Schienbein (13) unter der Haut eines Beins liegt und/oder
- ein Bereich mit einer geringeren Dicke (6.1, 6.2) als eine durchschnittliche Dicke der Hülle auf der Hülle liegt, wie ein Muskel (15) oder eine Sehne (16) unter der Haut eines Beins liegt,
wobei der Muskel oder die Sehne vorzugsweise der Solarmuskel (30), die anteriore Tibia (14), die lange Fibula (17), der lange Zehenextensor (18), der Gastroknemius (19) oder die Achillessehne (20) ist.

7. Hülle nach einem der vorhergehenden Ansprüche in Kombination mit Anspruch 2, wobei eine Außenfläche (21) der Hülle überwiegend glatt ist und die Dickenänderungen an einer Innenfläche (22) der Hülle liegen.

8. Hülle nach einem der vorhergehenden Ansprüche, wobei:
- ein Material der Hülle ein thermoplastisches Polyurethan (TPU) umfasst und/oder
- ein Material der Hülle ein Polyamid umfasst.

9. Baugruppe aus einer Verkleidungshülle für eine Beinprothese und einer Verkleidungshülle für eine Fußprothese,
Verkleidungshülle nach einem der Ansprüche 1 bis 8,
wobei die Verkleidungshülle der Beinprothese aus dem Material zusammen mit der Verkleidungshülle der Fußprothese besteht.

10. Baugruppe aus einer Verkleidungshülle für eine Prothese nach einem der Ansprüche 1 bis 8 und einer Hülle für einen Prothesenfuß (25), wobei die Verkleidungshülle eine röhrenförmige Form (23) aufweist und mindestens eine Nase (27) an einem unteren Rand (36) der röhrenförmigen Form umfasst, wobei die Nase reversibel in die Hülle des Prothesenfußes (25) einrasten kann, um sie demontierbar an der röhrenförmigen Form zu befestigen, vorzugsweise umfassend einen Ring (26) mit einer ersten Fläche und einer zweiten Fläche, wobei die erste Fläche des Rings die Nase (27) umfasst, wobei die zweite Fläche des Rings an dem unteren Rand (36) der röhrenförmigen Form befestigt, vorzugsweise angeklebt, ist.

11. Hülle nach einem der Ansprüche 1 bis 8,
wobei die Hülle einen Klemmkragen (28) umfasst, wobei der Kragen dazu geeignet ist, einen oberen Teil der röhrenförmigen Form auf einer Prothesenaufnahme (29) zu befestigen, um ein Gleiten zwischen der röhrenförmigen Form und der Aufnahme zu ermöglichen.

12. Baugruppe nach einem der Ansprüche 9 oder 10,
wobei die Hülle einen Klemmkragen (28) umfasst, wobei der Kragen dazu geeignet ist, einen oberen Teil der röhrenförmigen Form auf einer Prothesenaufnahme (29) zu befestigen, um ein Gleiten zwischen der röhrenförmigen Form und der Aufnahme zu ermöglichen.

13. Baugruppe aus einer Verkleidungshülle für eine Armprothese und einer Verkleidungshülle für eine Handprothese,
wobei die Verkleidungshülle nach einem der Ansprüche 1 bis 8 ist,
wobei die Verkleidungshülle der Armprothese aus dem Material zusammen mit der Verkleidungshülle der Handprothese besteht oder
wobei die Verkleidungshülle der Handprothese demontierbar an der Verkleidungshülle der Armprothese befestigt ist.

14. Verfahren zur Herstellung der Hülle nach einem der Ansprüche 1 bis 13, wobei das Verfahren die folgenden Schritte umfasst:
- Erhalten eines dreidimensionales Bildes einer Gliedmaße,
- Identifizieren, auf dem Bild, der Teile der Gliedmaße mit unterschiedlichen Härten (7, 8),
- Definieren verschiedener Flexibilitätsbereiche (5.1, 5.2, 6.1, 6.2) auf einem digitalen Modell der Hülle, so dass sie der Anordnung der auf dem Bild identifizierten Teile entsprechen,
- Herstellung der Hülle ausgehend von dem digitalen Modell, wobei vorzugsweise
- das dreidimensionale Bild von einem kompletten Bein mit dem Fuß oder von einem kompletten Arm mit der Hand erhalten wird,
- die Hülle die Hülle der Beinprothese und die Hülle der Fußprothese oder die Hülle der Armprothese und die Hülle der Handprothese umfasst, wobei das Verfahren vorzugsweise den folgenden Schritt umfasst:
- Identifizieren der Form der Gliedmaße auf dem Bild
- Definieren des digitalen Modells der Hülle, so dass die Form der Hülle der Form der Gliedmaße entspricht, wie auf dem Bild identifiziert.

15. Verfahren nach Anspruch 14, wobei der Ausführungsschritt ein 3D-Druckschritt, vorzugsweise durch Pulverschmelzen, der Hülle anhand des digitalen Modells ist.

## Claims

1. Covering shell (1) for a prosthesis (12) of a given limb (2), the shell comprising at least two zones (5.1, 5.2, 6.1, 6.2) of different flexibility (3.1, 3.2, 3.3, 9.1, 9.2),
Characterise din that :
the arrangement of said zones in relation to one another correspond to the arrangement of the parts of the given limb having different hardnesses (7, 8).

2. Shell according to claim 1, wherein a first zone (5.1, 5.2) differs from a second zone (6.1, 6.2) in relation to:
- a thickness and/or
- a material and/or
- a three-dimensional pattern
in order to obtain a first zone having a different flexibility from a second zone.

3. Shell according to claim 1 or 2, wherein:
- a first zone has a lesser flexibility (3.1, 3.2, 3.3) than a second zone, the first zone being arranged to coincide with a hard part (7) of the limb, and/or
- a second zone has a greater flexibility (9.1, 9.2) than a first zone, the second zone being arranged to coincide with a soft part (8) of the limb,
preferably wherein:
- the first zone (5.1., 5.2) and the second zone (6.1, 6.2) have different thicknesses, and
- the first zone and the second zone are a rib (10) or a recess (11).

4. Shell according to one of claims 1 to 3, wherein the shell has a tubular shape capable of receiving said prosthesis (12) inside the tubular shape,
preferably wherein :
- a flexibility of the shell remains mostly constant along a length of the tubular shape, and/or
- the flexibility of the shell mostly changes along a circumference of the tubular shape.

5. Shell according one of the preceding claims, the shell being adapted for housing a leg prosthesis, preferably a tibia prosthesis or an arm prosthesis, therein.

6. Shell according to claims 2 and 5 wherein:
- a zone having a greater thickness (3.1, 3.2, 3.3) than a mean thickness of the shell, the zone being located on the shell like the tibia (13) is located under a leg's skin and/or
- a zone having a lesser thickness (6.1, 6.2) than a mean thickness of the shell is located on the shell like a muscle (15) or a tendon (16) is located under a leg's skin,
the muscle or the tendon being preferably the soleus (30), the tibialis anterior (14), the fibularis longus (17), the extensor digitorum longus (18), the gastrocnemius (19) or Achille's tendon (20).

7. Shell according to one of the preceding claims in combination with claim 2, wherein an outer surface (21) of the shell is mostly smooth and the changes in thickness are located on an inner surface (22) of the shell.

8. Shell according to one of the preceding claims, wherein:
- a material of the shell comprises a thermoplastic polyurethane (TPU) and/or
- a material of the shell comprises a polyamide.

9. Assembly of a covering shell for a leg prosthesis and a covering shell for a foot prosthesis,
the covering shell being according to one of claims 1 to 8,
the covering shell of the leg prosthesis being integral with the covering shell of the foot prosthesis.

10. Assembly of a covering shell for a prosthesis according to one of claims 1 to 8 and a covering shell for a prosthetic foot (25), the covering shell having a tubular shape (23) and comprising at least one lug (27) on a bottom edge (36) of the tubular shape, the lug being capable of interlocking reversibly in the shell of the prosthetic foot (25), so as attach it removably to the tubular shape, preferably comprising a ring (26) with a first surface and a second surface, the first surface of the ring comprising the lug (27), the second surface of the ring being attached, preferably bonded, to the bottom edge (36) of the tubular shape.

11. Shell according to one of claims 1 to 8, wherein the shell comprises a gripping collet (28), said collet being capable of attaching an upper part of the tubular shape to a prosthesis socket (29) so as to enable sliding between the tubular shape and the socket.

12. Assembly according to one of claims 9 or 10, wherein the shell comprises a gripping collet (28), said collet being capable of attaching an upper part of the tubular shape to a prosthesis socket (29) so as to enable sliding between the tubular shape and the socket.

13. Assembly of a covering shell for an arm prosthesis and a covering shell for a hand prosthesis,
the covering shell being according to one of claims 1 to 8,
the covering shell of the arm prosthesis being integral with the covering shell of the hand prosthesis or
the covering shell of the hand prosthesis being removably attached to the covering shell of the arm prosthesis.

14. Method for manufacturing a shell according to one of claims 1 to 13, the method comprising the following steps:
- obtaining a three-dimensional image of a limb,
- identifying in the image the parts of the limb having different hardnesses (7, 8),
- defining on a digital model of the shell zones of different flexibility (5.1, 5.2, 6.1, 6.2) so as to correspond to the arrangement of said parts identified in said image,
- producing the shell from the digital model,
preferably wherein
- the three-dimensional image is obtained from a complete leg with its foot or from a complete arm with its hand,
- the shell comprises the shell of the leg prosthesis and the shell of the foot prosthesis or the shell of the arm prosthesis and the shell of the hand prosthesis,
the method preferably comprising the following step:
- identifying in the image the shape of the leg
- defining the digital model of the shell such that the shape of the shell corresponds to the shape of the limb as identified in the image.

15. Method according to claim 14, wherein the production step is a step of 3D printing, preferably using powder melting, of the shell from said digital model.
